# EUROPEAN PATENT APPLICATION

(11) **EP 4 678 013 A1**
(43) Date of publication of application: **14.01.2026**
(21) Application number: 24187230.8
(22) Date of filing: 08.07.2024
(51) Int. Cl.: A23L 33/17, A61K 38/17, A61P 7/06, C07K 14/805

(54) **NUTRITIONAL SUPPLEMENT COMPRISING MYOGLOBIN**

(71) Applicant: Paleo B.V., 3290 Diest (BE)
(72) Inventor: Sanctorum, Hermes, 3290 Diest (BE); de Jong, Andy, 3290 Diest (BE); Lauwers, Elsa, 3290 Diest (BE)
(74) Representative: Nederlandsch Octrooibureau

(57) **Abstract**

This invention relates to a nutritional supplement comprising a myoglobin and a method for preparing said nutritional supplement comprising a myoglobin, wherein the myoglobin may be a myoglobin obtained from fermentation by a microorganism.

## Description

### Field

This invention relates to a nutritional supplement comprising a myoglobin and a method for preparing said nutritional supplement comprising a myoglobin, wherein the myoglobin is obtained from fermentation by a microorganism which has been genetically modified to express the recombinant myoglobin.

### Background

It is well known that iron is crucial to the proper function of hemoglobin, which transports oxygen in the blood and perform other various processes. Lack of iron in the blood can lead to a range of symptoms, such as headache, dizziness, or serious health problems, including iron deficiency anemia.

Depending on the age, gender and physical situations, the amount of iron that one needs also varies. Even though iron is widely available in food, certain groups of people, such as adolescent girls and women ages 19 to 50 years old, or individuals recovering from surgery or blood disorders, or individuals with daily diet of insufficient iron source may not get the amount of iron they need from the daily diet.

A variety dietary supplements, including iron supplements, have been developed to help to get adequate amounts of essential nutrients if one does not get enough nutrients from daily food. However, many existing oral iron supplements (ferrous fumarate, ferrous gluconate, ferrous sulfate) have been shown to be associated with side effects such nausea and inefficient absorption. Iron may also decrease the absorption of other medications by forming an insoluble complex with those agents (i.e. methyldopa/levodopa, fluoroquinolones, penicillin, or tetracyclines).

Hence, there is need in the art for nutritional supplements which can provide bioavailable source of iron.

### Description

Proper nutrition is essential for maintaining health and preventing diseases. Adequate nutrition is especially critical during, for example, physiologically stressful periods. The nutritional supplement of the present invention may thus provide means to optimize, improve, or re-gain health by providing bioavailable source of myoglobin and iron. The nutritional supplement of the present invention may be administered to a subject such as a human.

In the first aspect of this invention, there is provided a nutritional supplement comprising a myoglobin for humans at risk for developing iron deficiency or an iron deficiency-related disorder.

A nutritional supplement is a product intended to provide additional nutritional value, preferably to a diet, to provide adequate intake of essential nutrients (preferably which are lacking in the diet), to enhance physical performance (such as athletic performance or recovery), to improve overall health and well-being, to prevent deficiencies (such as osteoporosis or anemia), and/or to support recovery from specific deficiencies. A nutritional supplement may be used to be added to a food product to transform said food product into a functional food or food product. Accordingly, the nutritional supplement itself is not a functional food or food product.

In the context of the present invention, a "nutritional supplement" may also mean a "dietary supplement", a "food supplement", a "health supplement", a "nutraceutical", or a "supplemental nutrition". Accordingly, these terms may be used interchangeably to describe products that provide additional nutrients to one's diet.

A nutritional supplement typically contains one or more of vitamins, minerals, amino acids, enzymes, herbs (or other botanicals), or other dietary substances such as fiber, fatty acids, or probiotics that contribute to the overall well-being. Vitamins are organic compounds essential for normal growth and nutrition. Minerals are inorganic elements that play a critical role in various physiological functions, such as calcium for bone health and iron for oxygen transport. Minerals may include calcium, phosphorus, magnesium, potassium, sodium, chloride, sulfur, iron, zinc, copper, manganese, iodine, selenium, fluoride, chromium, or molybdenum. Amino acids are organic compounds that combine to form proteins, which are crucial for muscle repair, enzyme production, and other vital functions. Enzymes are proteins that act as catalysts in biochemical reactions, aiding digestion, and metabolism.

A nutritional supplement is intended to complement a diet and may not be used to replace whole foods or diet. Accordingly, in the context of the present invention, the nutritional supplement is not a food product or part thereof. In some embodiments, the nutritional supplement is not a functional food or food product.

### 1. Myoglobin

Myoglobin is a relatively small globular protein of about 17 kDa, found in heart and skeletal muscles. It carries a single heme group with an atom of iron capable of reversible oxygen binding, allowing myoglobin to transport oxygen from the cell surface to mitochondria. Myoglobin contains 153 amino acids and as with other globins, consists of eight alpha helices connected by loops. Myoglobin contains a porphyrin ring with an iron at its center. A proximal histidine group (His-93) is attached directly to iron, and a distal histidine group (His-64) hovers near the opposite face. Myoglobin is an iron- and oxygen-binding protein found in the cardiac and skeletal muscle tissue of vertebrates in general and in almost all mammals. Muscle cells use myoglobin to accelerate oxygen diffusion and act as localized oxygen reserves for times of intense respiration.

In an embodiment, the nutritional supplement of the present invention comprises a myoglobin. In an embodiment, the myoglobin may be derived from steppe mammoth (Mammuthus trogontherii), woolly mammoth (Mammuthus primigenius), pig, sheep, cow, chicken, rabbit, bovine, mouse, rat, tuna, salmon, blue bonito, or sword fish. Myoglobin amino acid sequences from steppe mammoth, woolly mammoth, pig, sheep, cow, chicken, rabbit, bovine, mouse, rat, tuna, salmon, blue bonito, or sword fish have been disclosed later on by a given SEQ ID NO:1-14. In a preferred embodiment, the myoglobin may be derived from steppe mammoth (Mammuthus trogontherii) or woolly mammoth (Mammuthus primigenius).

In an embodiment, the myoglobin is derived from steppe mammoth, woolly mammoth, pig, sheep, cow, chicken, rabbit, bovine, mouse, rat, tuna, salmon, blue bonito, or sword fish by addition, insertion, deletion and/or substitution of at least one amino acid. Addition, insertion, deletion and/or substitutions of two, three, four, five, six, seven, eight, nine or ten amino acids is also contemplated by the invention.

In an embodiment, the myoglobin is a steppe mammoth myoglobin, woolly mammoth myoglobin, pig myoglobin, sheep myoglobin, cow myoglobin, chicken myoglobin, rabbit myoglobin, bovine myoglobin, mouse myoglobin, rat myoglobin, tuna myoglobin, salmon myoglobin, blue bonito myoglobin, or sword fish myoglobin or a myoglobin derived from any of these myoglobins.

In an embodiment, the myoglobin is steppe mammoth myoglobin (such as SEQ ID NO: 1) or derived therefrom. In an embodiment, the myoglobin is woolly mammoth myoglobin (such as SEQ ID NO: 2) or derived therefrom. In an embodiment, the myoglobin is pig myoglobin (such as SEQ ID NO: 3) or derived therefrom. In an embodiment, the myoglobin is sheep myoglobin (such as SEQ ID NO: 4) or derived therefrom. In an embodiment, the myoglobin is cow myoglobin (such as SEQ ID NO: 5) or derived therefrom. In an embodiment, the myoglobin is chicken myoglobin (such as SEQ ID NO: 6) or derived therefrom. In an embodiment, the myoglobin is rabbit myoglobin (such as SEQ ID NO: 7) or derived therefrom. In an embodiment, the myoglobin is bovine myoglobin (such as SEQ ID NO: 8) or derived therefrom. In an embodiment, the myoglobin is mouse myoglobin (such as SEQ ID NO: 9) or derived therefrom. In an embodiment, the myoglobin is rat myoglobin (such as SEQ ID NO: 10) or derived therefrom. In an embodiment, the myoglobin is tuna myoglobin (such as SEQ ID NO: 11) or derived therefrom. In an embodiment, the myoglobin is salmon myoglobin (such as SEQ ID NO: 12) or derived therefrom. In an embodiment, the myoglobin is blue bonito myoglobin (such as SEQ ID NO: 13) or derived therefrom. In an embodiment, the myoglobin is sword fish myoglobin (such as SEQ ID NO: 14) or derived therefrom.

In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with any one of the SEQ ID NOs: 1-14. In an embodiment, the myoglobin comprises a sequence having at least 95% identity with any one of SEQ ID NO: 1 to 14. In an embodiment, the myoglobin is represented by a sequence having at least 95% identity with any one of SEQ ID NO: 1 to 14.

In an embodiment, the myoglobin is steppe mammoth myoglobin (SEQ ID NO: 1). In an embodiment, the myoglobin is woolly mammoth myoglobin (SEQ ID NO: 2). In an embodiment, the myoglobin is pig myoglobin (SEQ ID NO: 3). In an embodiment, the myoglobin is sheep myoglobin (SEQ ID NO: 4). In an embodiment, the myoglobin is cow myoglobin (SEQ ID NO: 5). In an embodiment, the myoglobin is chicken myoglobin (SEQ ID NO: 6). In an embodiment, the myoglobin is rabbit myoglobin (SEQ ID NO: 7). In an embodiment, the myoglobin is bovine myoglobin (SEQ ID NO: 8). In an embodiment, the myoglobin is mouse myoglobin (SEQ ID NO: 9). In an embodiment, the myoglobin is rat myoglobin (SEQ ID NO: 10). In an embodiment, the myoglobin is tuna myoglobin (SEQ ID NO: 11). In an embodiment, the myoglobin is salmon myoglobin (SEQ ID NO: 12). In an embodiment, the myoglobin is blue bonito myoglobin (SEQ ID NO: 13). In an embodiment, the myoglobin is sword fish myoglobin (SEQ ID NO: 14).

In an embodiment, the myoglobin is derived from steppe mammoth myoglobin (SEQ ID NO: 1). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 1. A nucleic acid encoding said myoglobin which is derived from steppe mammoth myoglobin (SEQ ID NO:1) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:15.

In an embodiment, the myoglobin is derived from woolly mammoth myoglobin (SEQ ID NO: 2). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 2. A nucleic acid encoding said myoglobin which is derived from woolly mammoth myoglobin (SEQ ID NO: 2) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:16.

In an embodiment, the myoglobin is derived from pig myoglobin (SEQ ID NO: 3). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 3. A nucleic acid encoding said myoglobin which is derived from pig myoglobin (SEQ ID NO: 3) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:17.

In an embodiment, the myoglobin is derived from Sheep myoglobin (SEQ ID NO: 4). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 4. A nucleic acid encoding said myoglobin which is derived from Sheep myoglobin (SEQ ID NO: 4) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:18.

In an embodiment, the myoglobin is derived from cow myoglobin (SEQ ID NO: 5). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 5. A nucleic acid encoding said myoglobin which is derived from cow myoglobin (SEQ ID NO: 5) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:19.

In an embodiment, the myoglobin is derived from chicken myoglobin (SEQ ID NO: 6). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 6. A nucleic acid encoding said rmyoglobin which is derived from chicken myoglobin (SEQ ID NO: 6) may have a sequence that has at least 70%, at least 71 %, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:20.

In an embodiment, the myoglobin is derived from rabbit myoglobin (SEQ ID NO: 7). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 7. A nucleic acid encoding said myoglobin which is derived from rabbit myoglobin (SEQ ID NO: 7) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:21.

In an embodiment, the myoglobin is derived from bovine myoglobin (SEQ ID NO: 8). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 8. A nucleic acid encoding said myoglobin which is derived from bovine myoglobin (SEQ ID NO: 8) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:22.

In an embodiment, the myoglobin is derived from mouse myoglobin (SEQ ID NO: 9). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 9. A nucleic acid encoding said myoglobin which is derived from mouse myoglobin (SEQ ID NO: 9) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:23.

In an embodiment, the myoglobin is derived from rat myoglobin (SEQ ID NO: 10). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 10. A nucleic acid encoding said myoglobin which is derived from rat myoglobin (SEQ ID NO: 10) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:24.

In an embodiment, the myoglobin is derived from tuna myoglobin (SEQ ID NO: 11). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 11. A nucleic acid encoding said myoglobin which is derived from tuna myoglobin (SEQ ID NO: 11) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:25.

In an embodiment, the myoglobin is derived from salmon myoglobin (SEQ ID NO: 12). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 12. A nucleic acid encoding said myoglobin which is derived from salmon myoglobin (SEQ ID NO: 12) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:26.

In an embodiment, the myoglobin is derived from blue bonito myoglobin (SEQ ID NO: 13). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 13. A nucleic acid encoding said myoglobin which is derived from blue bonito myoglobin (SEQ ID NO: 13) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:27.

In an embodiment, the myoglobin is derived from sword fish myoglobin (SEQ ID NO: 14). In an embodiment, the myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 14. A nucleic acid encoding said myoglobin which is derived from sword fish myoglobin (SEQ ID NO: 14) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:28.

Myoglobins which may be represented by SEQ ID NO: 1-14 may be encoded by nucleic acids which may be represented by SEQ ID NO: 15-28, respectively. It is understood that the nucleic acids sequence encoding myoglobins or derivatives thereof as described above may need to be codon-optimized in order to be expressed by the microorganism or in order to optimize their production by the microorganism. In the context of the invention the myoglobin used may be considered as a recombinant protein.

In an embodiment, the myoglobin is a myoglobin obtained from microbial fermentation.

A "recombinant protein" may mean a protein, or a polypeptide encoded by a recombinant DNA, and produced by an expression system (or expression host or a microorganism), wherein said DNA is not endogenous or native for said expression system. DNA regulatory regions as promoters may also be non native for the expression system. Alternatively DNA regulatory regions such as promoters may be native for said expression system. In other words, the expression system has been transformed, introducing exogenous or foreign DNA into it. This expression system hence formed expresses said recombinant DNA that will be translated into a corresponding recombinant protein, during a production process such as a microbial fermentation process.

A microorganism used in the microbial fermentation may be a prokaryote, a eukaryote or a filamentous fungus. A prokaryote may be a bacterium. The bacterium may be a Gram positive/Gram negative bacterium slected from the following list: *Absidia, Achromobacter, Acinetobacter, Aeribacillus, Aneurinibacillus, Agrobacterium, Aeromonas, Alcaligenes, Arthrobacter, Arzoarcus, Azomonas, Azospirillum, Azotobacter, Bacillus, Beijerinckia, Bradyrhizobium, Brevibacills, Burkholderia, Byssochlamys, Citrobacter, Clostridium, Comamonas, Cupriavidus, Corynebacterium, Deinococcus, Escherichia, Enterobacter, Flavobacterium, Fusobacterium, Gossypium, Klebsiella, Lactobacillus, Listeria, Megasphaera, Micrococcus, Mycobacterium, Norcadia, Porphyromonas, Propionibacterium, Pseudomonas, Ralstonia, Rhizobium, Rhodopseudomonas, Rhodospirillum, Rodococcus, Roseburia, Shewanella, Streptomycetes, Xanthomonas, Xylella, Yersinia, Treponema, Vibrio, Streptococcus, Lactococcus, Zymomonas, Staphylococcus, Salmonella, Sphingomonas, Sphingobium, Novosphingobium, Brucella* and *Microscilla.* Preferred bacteria include *Aeribacillus pallidus, Aneurinibacillus terranovensis, Bacillus subtilis, Bacillus amyloliquefaciens, Bacillus coagulans, Bacillus licheniformis, Bacillus megaterium, Bacillus halodurans, Bacillus pumilus, Brevibacillus thermoruber, Brevibacillus panacihumi, Cupriavidus basilensis, G. Iraustophilus, Gluconobacter oxydans, Caulobacter crescentus CB 15, Methylobacterium extorquens, Rhodobacter sphaeroides, Pelotomaculum thermopropionicum, Pseudomonas zeaxanthinifaciens, Pseudomonas putida, Paracoccus denitrificans, Escherichia coli, Corynebacterium glutamicum, Staphylococcus carnosus, Streptomyces lividans, Sinorhizobium melioti, Sphingobium sp., Novosphingobium sp., Sphingomonas henshuiensis,* and *Rhizobium radiobacter.* A preferred bacterium is Escherichia coli. Preferred Escherichia coli strains include: 58, 679, WG1, DH5α, TG1, TOP10, K12, BL21, BL21 DE3, XL1-Blue, XL10-Gold, TB1, REG-12, W945, HB101, DH1, DP50, AB284, JC9387, AG1, C600, Cavalli Hfr, Y10.

A eukaryote may be a yeast or a filamentous fungus. Preferred yeasts include *Saccharomyces, Kluyveromyces, Candida, Pichia, Schizosaccharomyces, Hansenula, Kloeckera, Schwanniomyces, Yarrowia, Cryptococcus, Debaromyces, Saccharomycecopsis, Saccharomycodes, Wickerhamia, Debayomyces, Hanseniaspora, Ogataea, Kuraishia, Komagataella, Metschnikowia, Williopsis, Nakazawaea, Torulaspora, Bullera, Rhodotorula, Sporobolomyces.* Within yeasts, the species *Kluyveromyces lactis, Saccharomyces cerevisiae, Hansenula polymorpha* (also known as *Ogataea henricii), Yarrowia lipolytica, Candida tropicalis* and *Pichia pastoris* (also known as *Komagataella phaffii*) are preferred. Preferred Pichia strains are selected from the following list: Bg09, Bg10, Bg11, Bg12 (exemplified), Bg20, Bg21, Bg22, Bg23, Bg24, Bg25, Bg26, Bg40, Bg43, Bg44, Bg45, Y-11430, X-33, GS115, KM71, SMD1168, SMD1165, MC100-3, most preferred Bg10 and derivatives. Preferred Saccharomyces strains are selected from the following list: S288C, CEN.PK family, CBS 2354, ATCC 2360, ATCC 4098, ATCC 4124, ATCC 4126, ATCC 4127, ATCC 4921, ATCC 7754, ATCC 9763, ATCC 20598, ATCC 24855, ATCC 24858, ATCC 24860, ATCC 26422, ATCC 46523, ATCC 56069, ATCC 60222, ATCC 60223, ATCC 60493, ATCC 66348, ATCC 66349, ATCC 96581. A preferred yeast is a *Pichia* strain, more preferably *Pichia pastoris.*

A filamentous fungus may be selected from the following list including: *Acremonium, Agaricus, Aspergillus, Aureobasidium, Chrysosporium, Coprinus, Cryptococcus, Filibasidium, Fusarium, Humicola, Magnaporthe, Mucor, Myceliophthora, Neocallinastix, Neurospora, Paecilomyces, Penicillium, Piromyces, Panerochaete, Pleurotus, Schizophyllum, Talaromyces, Thermoascus, Thielavia, Tolypocladium, Ustilago and Trichoderma.* Preferred filamentous fungus are selected from the following list: *Aspergillus niger, Aspergillus nidulans, Aspergillus fumigatus, Aspergillus oryzae, Aspergillus vadensis, Penicillium chrysogenum, Penicillium citrinum, Penicillium rubens, Penicillium oxalicum, Penicillium subrubescens, Rasamsonia emersonii, Talaromyces emersonii, Acremonium chrysogenum, Trichoderma reesei, Aspergillus sojae,* and *Chrysosporium lucknowense.* Preferred strains of filamentous fungus are selected from the following list: *Aspergillus niger* CBS 513.88, N593, CBS 120.49, N402, ATCC 1015 *Aspergillus oryzae* ATCC 20423, IFO 4177, ATCC 1011, ATCC 9576, ATCC 14488-14491, ATCC 11601, ATCC12892, *Aspergillus vadensis* CBS 113365, CBS 102787, IMI 142717, IBT 24658, CBS 113226, *Penicillium chrysogenum* CBS 455.95, *Penicillium citrinum* ATCC 38065, *Penicillium chrysogenum* P2, Wisconsin 54-1255, *Penicillium subrubescens* CBS 132785, FBCC 1632, *Talaromyces emersonii* CBS 393.64, *Acremonium chrysogenum* ATCC 36225 or ATCC 48272, *Trichoderma reesei* ATCC 26921 or ATCC56765 or ATCC 26921, *Aspergillus sojae* ATCC11906, *Chrysosporium lucknowense* ATCC44006. In a preferred embodiment, *Aspergillus* is used as a filamentous fungus. More preferably, an *Aspergillus niger* strain is used.

In an embodiment, the microorganism used in the microbial fermentation may be a bacterium, a yeast, a filamentous fungus or a cultured mammalian cell line, preferably *Escherichia coli* or *Saccharomyces cerevisiae.* In this context, single, isolated, cultured mammalian cells may be considered as microorganisms. In a further embodiment, the microorganism may be a bacterium, a yeast or a filamentous fungus. The microorganisms in the context of this invention are useful for the production of a myoglobin, or a recombinant myoglobin.

The microbial fermentation comprises culturing the microorganisms in a suitable medium and optionally recovering the microorganism and/or the myoglobin, or recombinant myoglobin. Optionally, the produced myoglobin or recombinant myoglobin does not comprise a signal peptide as defined elsewhere herein. In an embodiment, the recovered myoglobin or recombinant myoglobin is purified. Preferably, the purified myoglobin or recombinant myoglobin has a purity of at least 70%, more preferably has a purity of at least 75%, 80%, 85%, 90%, 95%, 96%, 97%, 98%, 99%, most preferably has a purity of 100%.

In an embodiment, the myoglobin is extracellularly secreted from the microbial host, and/or wherein the myoglobin is recombinant myoglobin. In an embodiment, the microorganism produces the myoglobin or recombinant myoglobin extracellularly during the microbial fermentation. The myoglobin or recombinant myoglobin is transported out of the host cell after it is synthesized in the host cell, or the host microbial cell, or the host microorganism. In this context, both secretory and extracellular fermentations are considered to be extracellular productions. Without being bound to this theory, an extracellular production process has the advantage that the downstream processing to recover the produced myoglobin or recombinant myoglobin is more convenient, efficient and/or effective. Furthermore, an extracellular production process may result in a composition comprising the recombinant myoglobin with a high purity such as at least 60%, 65%, 70%, 75%, 80%, 85%, 90%, 95%, 96%, 97%, 98%, or 99% with minimal downstream processing compared to intracellular methods wherein the myoglobin or recombinant myoglobin is not transported out of the host cell after its synthesis. The relevant downstream processing technology that may be suitable for recovery and/or purification will depend on whether the myoglobin or recombinant myoglobin is accumulated within the cultured cells or excreted. Said processing technology and the associated choice will be known to the skilled person. Purity may be measured as the weight percentage of the total protein fraction in the cell-free supernatant obtained at the end of a process or extracellular process according to the invention. Without being bound to this theory, an extracellular production process has the advantage that the optional step of recovering myoglobin does not comprise lysing the host cell. As a result, the extracellular process may result in a composition having a low concentration of nucleic acids originating from the host cell. In an embodiment, the recovered myoglobin or recovered recombinant myoglobin is purified. Preferably, the purified myoglobin or purified recombinant myoglobin has a purity of at least 70%, more preferably has a purity of at least 75%, 80%, 85%, 90%, 95%, 96%, 97%, 98%, 99%, most preferably has a purity of 100%.

Optionally, the myoglobin or recombinant myoglobin is obtained via recovering and/or purifying from the cultured cells. A filter may be used for the purification of the recovered myoglobin. This may be realized continuously with the production process, by harvesting fractions of growing cells, or subsequently to it. In an embodiment, the myoglobin or recombinant myoglobin is isolated or obtained via recovering and/or purifying from the culture medium. This may be realized continuously with the production process or subsequently to it.

In some embodiments, the nutritional supplement according to the present invention comprises at least part of the fermentation broth or part of the microbial host.

In an embodiment, the nutritional supplement comprising a myoglobin as provided by the present invention still comprises at least part of the fermentation broth from the microbial fermentation as described herein or part thereof. "Fermentation broth" refers to the liquid medium or cell culture medium in which microorganisms used in the microbial fermentation are cultured to produce the myoglobin as described herein. Fermentation broth may contain at least part of the microbial host used to produce the myoglobin. Within this context, "par thereof" or "at least part of" may mean at least 20%, 30%, 40%, 50%, 60%, 70%, 80%, 90% of the initial mass amount (of the fermentation broth or of the microbial host) present at the end of the fermentation process. In an embodiment, the (total) weight concentration of the fermentation broth in the nutritional supplement is at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, at least 20%. In an embodiment, the weight fraction of myoglobin in the fermentation broch in the nutritional supplement is at least 5%, 10%, 15%, 20%, 25%, 30%, 35%, 40%, 45%, or 50%.

In an embodiment, the nutritional supplement comprising a myoglobin as provided by the present invention comprises at least part of the microorganisms used in the microbial fermentation as described herein. In an embodiment, the microorganism is not alive or viable. In an embodiment, the microorganism is not intact. In this context, "intact" may mean that the microorganism is lysed, or part of the microorganism is obtained. In an embodiment, the microorganism is yeast. In an embodiment, the (total) weight concentration of the yeast in the nutritional supplement is at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, at least 20%. In an embodiment, the weight fraction of hemeprotein in the fermentation yeast in the nutritional supplement is at least 5%, 10%, 15%, 20%, 25%, 30%, 35%, 40%, 45%, or 50%. In an embodiment, the yeast is *Pichia pastoris.*

In an embodiment, the nutritional supplement comprising a myoglobin as provided by the present invention comprises yeast extract, wherein the yeast is used as the microorganisms in the microbial fermentation. In an embodiment, the (total) weight concentration of the yeast extract in the nutritional supplement is at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, at least 20%. A "yeast extract" refers to yeast cell contents without cell walls. Typically, yeast extracts are produced from steps of: fermentation (growing the yeast), disruption (breaking of the cells), and separation (to keep the soluble part of the yeast cellular content). In an embodiment, the weight fraction of hemeprotein in the yeast extract in the nutritional supplement is at least 5%, 10%, 15%, 20%, 25%, 30%, 35%, 40%, 45%, or 50%. In an embodiment, the yeast is *Pichia pastoris,* and the yeast extract is *Pichia pastoris* extract.

All these previously defined embodiments comprising either the fermentation broth, the microorganism host, the culture medium and/or yeast extract are advantageous. Nutritional supplements comprising any of these elements in parallel to the myoglobin defined herein are expected to exhibit improved properties in terms of amount of bioavailable source of iron and/or in terms of amounts of essential nutrients.

In an embodiment, the nutritional supplement of the present invention comprises iron in the form of myoglobin-iron complex. Myoglobin is a relatively small globular protein of about 17 kD, found in heart and skeletal muscles. It carries a single heme group with an atom of iron capable of reversible oxygen binding allowing myoglobin to transport oxygen from the cell surface to mitochondria.

In an embodiment, the nutritional supplement of the present invention comprises 0.5 mg to 40 mg, 0.5 mg to 39 mg, 0.5 mg to 38 mg, 0.5 mg to 37 mg, 0.5 mg to 36 mg, 0.5 mg to 35 mg, 0.5 mg to 34 mg, 0.5 mg to 33 mg, 0.5 mg to 32 mg, 0.5 mg to 31 mg, 0.5 mg to 30 mg, 0.5 mg to 29 mg, 0.5 mg to 28 mg, 0.5 mg to 27 mg, 0.5 mg to 26 mg, 0.5 mg to 25 mg, 0.5 mg to 24 mg, 0.5 mg to 23 mg, 0.5 mg to 22 mg, 0.5 mg to 21 mg, 0.5 mg to 20 mg, 0.5 mg to 19 mg, 0.5 mg to 18 mg, 1 mg to 18 mg, 2 mg to 18 mg, 3 mg to 18 mg, 4 mg to 18 mg, 5 mg to 18 mg, 6 mg to 18 mg, 7 mg to 18 mg, 8 mg to 18 mg, 9 mg to 18 mg, 10 mg to 18 mg, 11 mg to 18 mg, 12 mg to 18 mg, 13 mg to 18 mg, 14 mg to 18 mg, or 15 mg to 18 mg of iron expressed as myoglobin per 100 mg of said nutritional supplement. In this context, the term "iron" may be used interchangeably with the term "Fe". "Expressed as myoglobin" in this context means that the iron or Fe content is sourced from the myoglobin as described herein. "Sourced from" may mean that the iron or Fe is complexed to or bound to the myoglobin comprised in the nutritional supplement of the present invention as described herein. In other word, the iron or Fe is contained within the myoglobin comprised in the nutritional supplement of the present invention. In an embodiment, the nutritional supplement of the present invention comprises 0.01 mg to 18 mg of iron expressed as myoglobin.

In an embodiment, the nutritional supplement of the present invention may further comprise iron or Fe content that is not sourced from the myoglobin as described herein or is not contained within the myoglobin in a form of complex with said myoglobin. In an embodiment, the nutritional supplement of the present invention may comprise 0.01 mg to 40 mg, 0.01 mg to 39 mg, 0.01 mg to 38 mg, 0.01 mg to 37 mg, 0.01 mg to 36 mg, 0.01 mg to 35 mg, 0.01 mg to 34 mg, 0.01 mg to 33 mg, 0.01 mg to 32 mg, 0.01 mg to 31 mg, 0.01 mg to 30 mg, 0.01 mg to 29 mg, 0.01 mg to 28 mg, 0.01 mg to 27 mg, 0.01 mg to 26 mg, 0.01 mg to 25 mg, 0.01 mg to 24 mg, 0.01 mg to 23 mg, 0.01 mg to 22 mg, 0.01 mg to 21 mg, 0.01 mg to 20 mg, 0.01 mg to 19 mg, 0.01 mg to 18 mg, 0.05 mg to 18 mg, 0.1 mg to 18 mg, 0.2 mg to 18 mg, 0.5 mg to 18 mg, 1 mg to 18 mg, 2 mg to 18 mg, 3 mg to 18 mg, 4 mg to 18 mg, 5 mg to 18 mg, 6 mg to 18 mg, 7 mg to 18 mg, 8 mg to 18 mg, 9 mg to 18 mg, 10 mg to 18 mg, 11 mg to 18 mg, 12 mg to 18 mg, 13 mg to 18 mg, 14 mg to 18 mg, or 15 mg to 18 mg of iron that is not expressed as myoglobin or that is not contained within the myoglobin as described herein. Some non-limiting examples of iron source that is not expressed as myoglobin may be a ferrous fumarate, a ferrous gluconate, or a ferrous sulfate, or others known to a skilled person in the art.

In an embodiment, the nutritional supplement of the present invention comprises at least 0.01 % by weight of the myoglobin as described herein. In an embodiment, the nutritional supplement of the present invention comprises from 0.01% up to 5%, 4.9%, 4.8%, 4.7%, 4.6%, 4.5%, 4.4%, 4.3%, 4.2%, 4.1%, 4%, 3.9%, 3.8%, 3.7%, 3.6%, 3.5%, 3.4%, 3.3%, 3.2%, 3.1%, 3%, 2.95%, 2.9%, 2.85%, 2.8%, 2.75%, 2.7%, 2.65%, 2.6%, 2.55%, 2.5%, 2.45%, 2.4%, 2.35%, 2.3%, 2.25%, 2.2%, 2.15%, 2.1%, 2.05%, 2%, 1.95%, 1.9%, 1.85%, 1.8%, 1.75%, 1.7%, 1.65%, 1.6%, 1.55%, 1.5%, 1.45%, 1.4%, 1.35%, 1.3%, 1.25%, 1.2%, 1.15%, 1.1%, 1.05%, 1%, 0.95%, 0.9%, 0.85%, 0.8%, 0.75%, 0.7%, 0.65%, 0.6%, 0.55%, 0.5%, 0.4%, 0.3%, 0.2%, or 0.1% by weight of the myoglobin as described herein.

In an embodiment, the nutritional supplement of the present invention may further comprise multiple vitamins, minerals and/or nutrients. Non limiting examples of said multiple vitamins, minerals and/or nutrients include vitamin A, beta-carotene alone, carotenoids, lycopene, lutein, zeaxanthin, cryptoxanthin, thiamine (vitamin B1), riboflavin (vitamin B2), niacin (vitamin B3), pantothenic acid (vitamin B5), pyridoxine (vitamin B1), folate (vitamin B9), cyanocobalamin (vitamin B12), vitamin C-complex, vitamin D, vitamin E, tocopherols, tocotrieneols, iodine, , iron, zinc, copper, magnesium, and/or omega 3 fatty acids. Each of the ingredients described herein may be combined in intimate admixture with a suitable carrier according to conventional compounding techniques known to the skilled person. The carrier may take a wide variety of forms depending upon the form of the preparation desired for oral administration.

In some embodiments, the concentration of an ingredient in the nutritional supplement, such as vitamins, minerals, amino acids, enzymes, herb, or other dietary substances as described above is higher than the corresponding ingredient in a food product. In an embodiment, the concentration of an ingredient in the nutritional supplement is at least 10%, at least 20%, at least 30%, at least 40%, at least 50%, at least 60%, at least 70%, at least 80%, at least 90%, at least 100%, at least 200%, at least 300%, at least 400% higher, at least 500% higher, at least 1000% higher, or at least 2000% higher, or at least 3000% higher, or at least 4000%, higher or at least 5000% higher than the corresponding ingredient in a food product. In this context, a food product may be natural food product or raw food product (such as fruits, vegetables, grains, meat, seafood or dairy product), processed food product (including snacks such as chips, beverages such as beer or juice, preserved food such as canned food, and frozen food such as pizza or ice cream), prepared meals (including meals made from a meal kit), or bakery and confectionery (including bread, cakes or chocolates).

In some embodiments, said ingredients, such as vitamins (i.e. vitamin B), minerals, amino acids, enzymes, or proteins are sourced from the fermentation broth and comprised in the nutritional supplement as described herein. In some embodiments, said ingredients, such as vitamins (i.e. vitamin B), minerals, amino acids, enzymes, or proteins are sourced from the microbial culture medium, preferably yeast culture medium and comprised in the nutritional supplement as described herein. In some embodiments, said ingredients, such as vitamins (i.e. vitamin B), minerals, amino acids, enzymes, or proteins are sourced from the yeast extract and comprised in the nutritional supplement as described herein.

In an embodiment, the nutritional supplement according to the present invention is formulated as edible, preferably comprises an edible carrier. In an embodiment, the nutritional supplement according to the present invention is formulated for oral administration. In preparing the composition in edible form, or optionally oral dosage form for oral administration, any of the usual media may be utilized. For liquid preparations (e.g., suspensions, elixirs, and solutions), media containing, for example water, oils, alcohols, flavouring agents, preservatives, colouring agents and the like may be used. Pharmaceutical acceptable carriers such as starches, sugars, diluents, granulating agents, lubricants, binders, disintegrating agents and the like may be used to prepare oral solids (e.g., powders, caplets, pills, tablets, capsules, and lozenges). Controlled release forms may also be used. Caplets, tablets, pills, and capsules represent the most advantageous oral dosage unit form, in which case solid carriers are employed. If desired, tablets may be sugar coated or enteric coated by standard techniques. Pharmaceutical carriers and formulations are well known to those of ordinary skill in the art.

In an embodiment, the nutritional supplement of the present invention may be formulated as solid or liquid form. In an embodiment, the nutritional supplement of the present invention may be formulated as a capsule, tablet, caplet, gel caplet (gelcap), syrup, a liquid composition, a liquid solution, a liquid suspension, a powder, a concentrated powder, a concentrated powder admixed with a liquid, a chewable form, a swallowable form, a dissolvable form, a biodegradable form, an effervescent, a granulated form, and an oral liquid solution. In an embodiment, the nutritional supplement according to the present invention is formulated as a powder, a capsule, a tablet, a chewable, an extruded bar, or a liquid solution or suspension.

In an embodiment, the nutritional supplement of the present invention may be formulated as a gelcap. In an embodiment, the gelcap may be a liquid gelcap which may consist of a filler comprising one or more pharmaceutically active materials dissolved or dispersed in an appropriate liquid vehicle encapsulated in a gelatine shell generally comprising gelatine together with a plasticizer such as glycerine or sorbitol. The filler material may comprise, for example, polyethylene glycols. A liquid gelcap has advantages of consumer acceptance and is easier to swallow due to the outer coating being a soft and elastic gelatine shell. Also, liquid compositions are well suited for encapsulation within a soft gelatine shell, creating flexibility that further assists in the capsule being easier to swallow.

In an embodiment, the gelcap may be a soft-gel gelcap. A soft-gel is a one-piece, sealed, soft gelatine shell that contains a solution, a suspension, or a semi-solid paste. Because soft-gels have properties that are quite different from two-piece, hard shell capsules, the soft-gels are capable of retaining a liquid fill material. Soft-gels are often used to encapsulate consumable materials, including vitamins, dietary supplements, pharmaceuticals, and the like, in a liquid vehicle or carrier. Soft-gels arc a unique dosage form that can provide distinct advantages over more traditional dosage forms such as tablets, hard-shell capsules, and liquids. These advantages include patient compliance and consumer preference, improved bioavailability, speed of product development in many cases, shortened manufacturing time, enhanced drug stability due to less exposure of the active ingredient to oxygen, excellent dose uniformity, and product differentiation.

"Chewable form" refers to solid but relatively soft nutritional supplements that are chewed in the mouth after oral administration, which may have a pleasant taste and mouthfeel, and may quickly break into smaller pieces and may begin to dissolve after chewing such that they can be swallowed substantially as a solution. Chewable nutritional supplement may include ingredients that create pleasant flavour and mouthfeel and promote relative softness and dissolvability in the mouth. A variety of ingredients can be included in the compositions of the present invention to enhance mouthfeel. For example, sugars such as white sugar, corn syrup, sorbitol (solution), maltitol (syrup), oligosaccharide, isomaltooligosaccharide, sucrose, fructose, lactose, glucose, lycasin, xylitol, lactitol, erythritol, mannitol, isomaltose, dextrose, polydextrose, dextrin, compressible cellulose, compressible honey, compressible molasses and mixtures thereof may be added to improve mouthfeel and palatability. For example fondant or gums such as gelatin, agar, arabic gum, guar gum, and carrageenan may be added to improve the chewiness of the nutritional supplements. For example, fatty materials such as vegetable oils (including palm oil, palm hydrogenated oil, corn germ hydrogenated oil, castor hydrogenated oil, cotton-seed oil, olive oil, peanut oil, palm olein oil, and palm stearin oil), animal oils, cacao fat, margarine, or butter may be included in the nutritional supplements of the present invention. "Enhance the mouthfeel" means that the gritty texture of the nutritional supplement is reduced once it has liquefied in the mouth, relative to the same preparation lacking the corresponding ingredient(s). "Enhance the texture" means that one or more of the stiffness, the brittleness, and the chewiness of the chewable nutritional supplement has been improved relative to the same preparation lacking corresponding ingredient(s).

In an embodiment, Alkyl polysiloxanes may be used in the chewable nutritional supplement of the present invention to enhance the texture, and/or the mouthfeel. Exemplary alkyl polysiloxanes monoalkyl or dialkyl polysiloxanes. In an embodiment, the Alkyl polysiloxane is dimethyl polysiloxane (or simethicone). In an embodiment, the Alkyl polysiloxane is a granular simethicone.

In an embodiment, the chewable nutritional supplement of the present invention may include rapidly water-soluble fillers and excipients. Examples of rapidly water-soluble fillers include saccharides, amino acids and the like.

In an embodiment, the chewable nutritional supplement of the present invention may include disintegrants. Said disintegrants may include corn starch, potato starch, pre-gelatinized and modified starches thereof, cellulosic agents, such as Ac-di-sol, montrnorrilonite clays, cross-linked PVP, sweeteners, bentonite, microcrystalline cellulose, croscarmellose sodium, alginates, sodium starch glycolate, gums, such as agar, guar, locust bean, karaya, pectin, Arabic, xanthan and tragacanth, silica with a high affinity for aqueous solvents, such as colloidal silica, precipitated silica, maltodextrins, beta-cyclodextrins, polymers, such as carbopol, and cellulosic agents, such as hydroxymethylcellulose, hydroxypropylcellulose and hydroxyopropylmethylcellulose.

"Dissolvable form" refers to formulations that dissolve into a solution in the mouth. Such nutritional supplements in dissolvable form may dissolve within about 60 seconds or less after placement in the mouth without any chewing. Dissolution of the nutritional supplement of the present invention may be facilitated by including relatively small particles sizes of the ingredients used.

"Biodegradable form" refers to formulations that are degradable through natural processes involving microorganisms such as bacteria, fungi, human beings or other biological means. A "biodegradable nutrient supplement" may refer to a type of nutritional product designed to provide essential nutrients, such as vitamins, minerals, or other to support health and well-being, which is made from materials that can break down naturally and safely in the human gut and/or in the environment.

The term "mouthfeel" refers to non-taste-related aspects of the pleasantness experienced by a person while chewing or swallowing a nutritional supplement. Aspects of mouthfeel include, for example and without limitation, the hardness and brittleness of a nutritional supplement, whether the nutritional supplement is chewy, gritty, oily, creamy, watery, sticky, easily dissolved, astringent, effervescent, and the like, and the size, shape, and form of the nutritional supplement (tablet, powder, gel, etc.).

The phrase "swallowable form" refers to any nutritional supplement that typically do not or are not configured to readily dissolve when placed in the mouth and may be swallowed whole, preferably without chewing or discomfort. Such nutritional supplements may have a shape containing no sharp edges and a smooth, uniform and substantially bubble free outer coating. In an embodiment, the swallowable nutritional supplement may have a shape containing no sharp edges and a smooth, uniform and substantially bubble free outer coating. In an embodiment, the surface of the swallowable nutritional supplement may be coated with a polymeric film. Such a film coating may reduce the adhesion of the nutritional supplement to the inner surface of the mouth, thereby increasing the subject's ability to swallow the nutritional supplement, aid in masking the taste of the swallowable nutritional supplement or may protect the swallowable nutritional supplement from atmospheric degradation. Said polymeric films may include vinyl polymers such as polyvinylpyrrolidone, polyvinyl alcohol and acetate, cellulosics such as methyl and ethyl cellulose, hydroxyethyl cellulose and hydroxylpropyl methylcellulose, acrylates and methacrylates, copolymers such as the vinyl-maleic acid and styrenemaleic acid types, and natural gums and resins such as zein, gelatin, shellac and acacia.

In an embodiment, the nutritional supplement according to the present invention further comprises one or more food-grade additives selected from the group comprising: mixing aids, emulsifiers, sweeteners, preservatives, colorants, and flavour additives.

In an embodiment, the nutritional supplement according to the present invention may comprise binders. Such binders may include acacia, compressible sugar, gelatin, sucrose and its derivatives, maltodextrin, cellulosic polymers, such as ethylcellulose, hydroxypropylcellulose, hydroxypropylmethyl cellulose, carboxymethylcellulose sodium and methylcellulose, acrylic polymers, such as insoluble acrylate ammoniomethacrylate copolymer, polyacrylate or polymethacrylic copolymer, povidones, copovidones, polyvinylalcohols, alginic acid, sodium alginate, starch, pregelatinized starch, guar gum, polyethylene glycol and others known to the skilled person in the art.

In an embodiment, the nutritional supplement according to the present invention may comprise diluents. Said diluents may include microcrystalline cellulose, sucrose, dicalcium phosphate, starches, lactose and polyols of less than 13 carbon atoms, such as mannitol, xylitol, sorbitol, maltitol and pharmaceutically acceptable amino acids, such as glycin, and their mixtures.

In an embodiment, the nutritional supplement according to the present invention may comprise Lubricants. Said lubricants may include stearic acid, calcium stearate, magnesium stearate, zinc stearate, talc, mineral and vegetable oils, benzoic acid, polyethylene glycol), glyceryl behenate, stearyl fumarate, and others known to the skilled person in the art.

In an embodiment, the nutritional supplement according to the present invention may comprise glidants. Glidants may include silicon dioxide, colloidal or fumed silica, magnesium stearate, calcium stearate, stearic acid, cornstarch, talc and others known to those of ordinary skill in the art.

In an embodiment, the nutritional supplement according to the present invention may comprise colorants. Said colorants may include FD&C Red No. 3, FD&C Red No. 20, FD&C Yellow No. 6, FD&C Blue No. 2, D&C Green No. 5, FD&C Orange No. 5, D&C Red No. 8, caramel, and ferric oxide, red and others known to the skilled person in the art. Coloring agents may include pigments, dyes, tints, titanium dioxide, natural coloring agents, such as grape skin extract, beet red powder, beta carotene, annato, carmine, tumeric, paprika and others known to the skilled person in the art.

The nutritional supplement of the present invention may be sugar coated or enteric coated by standard techniques known to the skilled person in the art. The unit dose forms may be individually wrapped, packaged as multiple units on paper strips or in vials of any size, without limitation. The swallowable, chewable or dissolvable nutritional supplement of the present invention may be packaged in unit dose, rolls, bulk bottles, blister packs and combinations thereof, without limitation.

### 2. Iron deficiency

Iron balance is essential for life. A primary function of iron is to carry oxygen to bodily tissues via the hemoglobin part of red blood cells. Iron homeostatic mechanisms evolved to avoid iron excess and the generation of harmful reactive oxygen species by reutilizing body iron and limiting its uptake from the environment. Supplemental intake of iron is critical to preventing iron deficiency associated syndromes or disorders, such anemia, a disorder associated with a variety of physiological states including, for example, pregnancy. Other iron deficiency associated syndromes or disorders include IDA (iron deficiency anemia), anorexia, syndrome of heavy menstrual bleeding (HMB), pica, or restless legs syndrome (RLS).

Iron deficiency is the depletion of total-body iron, especially of macrophage and hepatocyte iron stores. Iron deficiency is a broader condition that often precedes the onset of anemia or indicates deficiency in organs/tissues other than those involved in erythropoiesis, such as skeletal muscles and the heart.

Because the largest amount of iron is consumed for hemoglobin (Hb) synthesis to produce 200 billion erythrocytes daily, anemia is the more evident sign of iron deficiency, and iron deficiency anemia is often considered synonymous with iron deficiency.

Iron deficiency anemia is a common type of anemia, which is a condition in which blood lacks adequate healthy red blood cells. Anemia may be defined as when the hemoglobin level in blood less than 13 g/dL in males, or less than 12 g/dL in females, or less than 11g/dL during pregnancy, according to the World Health Organization recommendation. Symptoms of iron deficiency anemia may include extreme fatigue, weakness, paleness in the skin, chest pain, fast heartbeat or shortness of breath, headache, dizziness or lightheadedness, cold hands and feet, inflammation or soreness of your tongue, brittle nails, unusual cravings for non-nutritive substances, such as ice, dirt or starch, and poor appetite, especially in infants and children with iron deficiency anemia.

**Table 1. Main causes of absolute iron deficiency/iron deficiency anemia**

| **Type of cause** | **Condition** | **Pathophysiologic mechanism** |
|---|---|---|
| Increased iron requirements | Infants, preschool children, adolescents | Rapid growth |
| | Pregnant women: second and third trimesters | Expansion of maternal and fetal erythroid mass |
| | ESA treatment | Acute expansion of erythroid mass |
| Low iron intake | Malnutrition | Insufficient dietary iron: low heme iron or scarcely bioavailable iron (eg, chelated by phytates) |
| | Vegetarians, vegans | |
| Decreased intestinal iron absorption | Gastrectomy, duodenal bypass, bariatric surgery | Decreased absorptive surface |
| | Gluten-induced enteropathy | |
| | Autoimmune atrophic gastritis | Increased pH |
| | *Helicobacter pylori* infection | Increased pH and blood loss |
| | Drugs: proton pump inhibitors, H₂ blockers | Blocking of gastric acid secretion |
| | Genetic IRIDA | High serum hepcidin levels |
| Chronic blood loss | Hookworm infestation | Bleeding from gastrointestinal tract |
| | Gastrointestinal benign and malignant lesions | |
| | Salicylates, corticosteroids, nonsteroidal anti-inflammatory drugs | |
| | Heavy menses, hematuria | Bleeding from genitourinary system |
| | Intravascular hemolysis (PNH, march hemoglobinuria) | Urinary loss of hemoglobin (iron) |
| | Drugs: anticoagulants, antiplatelet compounds | Systemic bleeding |
| | Defects of hemostasis (hereditary hemorrhagic telangectasia, von Willebrand disease) | |
| | Frequent blood donors | Repeated blood letting |
| Multiple causes (absolute iron deficiency associated with inflammation) | Chronic infections in malnutrition | Reduced intake, increased proinflammatory cytokines |
| | Chronic kidney disease | Decreased iron absorption, increased blood loss, reduced hepcidin excretion and increased production, drugs, ESAs |
| | Chronic systolic heart failure | Decreased iron absorption, increased inflammation, blood loss |
| | Inflammatory bowel diseases | Decreased iron absorption, increased blood loss, high hepcidin |
| | Postoperative anemia of major surgery | Blood loss, increased proinflammatory cytokines |

Iron deficiency or iron deficiency anemia may be tested, examined, diagnosed or detected by diagnostic tests known to the skilled person. Non-limiting examples include full blood examination, measuring serum ferritin levels, or measuring Transferrin and transferrin saturation. In full blood examination, Haemoglobin (Hb) level is used to determine the presence and severity of anaemia. Anaemia is defined as a haemoglobin (Hb) concentration below the reference range for the laboratory performing the test, or below the WHO cut-off levels as described above. Low serum ferritin level can be used as a hallmark of absolute iron deficiency, reflecting exhausted stores. For example, a human subject having a serum ferritin level of lower than 30 mg/L may be identified as having mild "iron deficiency". A human subject having a serum ferritin level of lower than 10-12 mg/L may be identified as having anemia or "iron deficiency anemia". Elevated transferrin and low transferrin saturation are suggestive of iron deficiency even in the presence of a normal or elevated ferritin. A transferrin saturation of < 20% may be used to define low iron availability in both absolute and functional iron deficiency.

In an embodiment, the nutritional supplement of the present invention may be used as a dietary supplement for humans having iron deficiency or an iron deficiency-related disorder. In another embodiment, the nutritional supplement of the present invention may be used as a prescription dietary supplement.

In another embodiment, the nutritional supplement of the present invention, may be used as a medicament.

In an embodiment, the nutritional supplement of the present invention may further comprise a pharmaceutically acceptable carrier. The phrase "pharmaceutically acceptable," as used herein, refers to those compounds, materials, compositions and/or dosage forms which are suitable for use in contact with the tissues of human beings without excessive toxicity, irritation, allergic response, or other problem or complication, commensurate with a reasonable benefit/risk ratio. Thus, the phrase "pharmaceutically acceptable carriers," as used herein, refers to such suitable compounds and materials defined above that may be added to the dosage form to assist in satisfactory processing of the dosage form or provide desirable physical characteristics to the dosage form. For example, "pharmaceutically acceptable carriers" may include, but is not limited to, binders, diluents, lubricants, glidants, colorants, emulsifiers, disintegrants, starches, water, oils, alcohols, preservatives, and sugars. In addition, the pharmaceutical composition or formulation may also include other carriers, or non-toxic, nontherapeutic, non-immunogenic stabilizers and the like.

"Pharmaceutically acceptable carriers" for dosage forms such as capsules, caplets, or gel-caps may comprise omega-3 fatty acids such as docosahexaenoic acid (DHA).

Examples of diluents are distilled water, physiological phosphate-buffered saline, Ringer's solutions, dextrose solution, and Hank's solution.

Examples of suitable aqueous and non-aqueous pharmaceutically acceptable carriers include water, saline, phosphate-buffered saline, ethanol, dextrose, polyols (such as glycerol, propylene glycol, polyethylene glycol, and the like), and suitable mixtures thereof, vegetable oils, such as olive oil, corn oil, peanut oil, cottonseed oil, and sesame oil, carboxymethyl cellulose colloidal solutions, tragacanth gum and injectable organic esters, such as ethyl oleate, and/or various buffers. Other carriers are well-known in the pharmaceutical arts.

The pharmaceutically acceptable carriers may also comprise antioxidants including water soluble antioxidants, such as ascorbic acid, cysteine hydrochloride, sodium bisulfate, sodium metabisulfite, sodium sulfite and the like; oil-soluble antioxidants, such as ascorbyl palmitate, butylated hydroxyanisole (BHA), butylated hydroxytoluene (BHT), lecithin, propyl gallate, alpha- tocopherol, and the like; and metal chelating agents, such as citric acid, ethylenediamine tetraacetic acid (EDTA), sorbitol, tartaric acid, phosphoric acid, and the like.

In an embodiment, the nutritional supplement of the present invention may be used as a medicament. In an embodiment, said nutritional supplement may further comprise a pharmaceutically acceptable carrier.

Symptoms of iron deficiency anemia may include extreme fatigue, weakness, paleness in the skin, chest pain, fast heartbeat or shortness of breath, headache, dizziness or lightheadedness, cold hands and feet, inflammation or soreness of your tongue, brittle nails, unusual cravings for non-nutritive substances, such as ice, dirt or starch, and poor appetite, especially in infants and children with iron deficiency anemia.

In an embodiment, the nutritional supplement of the present invention may be for use in the treatment of iron deficiency, or an iron deficiency-related disorder as defined herein. In an embodiment, the iron deficiency-related disorder is iron deficiency anaemia.

In an embodiment, the nutritional supplement of the present invention for use in the treatment of iron deficiency or an iron deficiency-related disorder as described above, is able to delay, prevent, reduce or reverse one or more of the following symptoms: extreme fatigue, weakness, paleness in the skin, chest pain, fast heartbeat or shortness of breath, headache, dizziness or lightheadedness, cold hands and feet, inflammation or soreness of your tongue, brittle nails, unusual cravings for non-nutritive substances, such as ice, dirt or starch, and poor appetite. The symptoms may be assessed using techniques known to a skilled person. The delay, prevention, reduction, or reversion of a symptom may mean that an improvement of at least 10%, 20%, 30%, 40%, 50%, 60%, 70% has been determined compared to the measurements of the same symptom in a control subject having iron deficiency or iron deficiency related disorder in the absence of administration of the nutritional supplement of the present invention (or in the subject treated with the nutritional supplement of the present invention at the onset of the treatment). The improvement of a symptom may also mean that an improvement of at least 60%, 70%, 80% or 90% has been determined compared to the measurement in a control/healthy subject without iron deficiency or iron deficiency related disorder (such as iron deficiency anemia). The delay, prevention, reduction, or reversion of one or more the symptoms as described above is attributable to the myoglobin, the recombinant myoglobin, and/or the iron content in the myoglobin present in the nutritional supplement of the present invention.

The term "treatment" or "treating" as used herein means ameliorating, slowing or reversing the progress or severity of a disease or disorder, or ameliorating, slowing or reversing one or more symptoms or side effects of such disease or disorder. The term "delaying" as used herein means slowing the progress or severity of a disease or disorder, or ameliorating, slowing one or more symptoms or side effects of such disease or disorder. The term "reversing" as used herein means reversing the progress or severity of a disease or disorder or ameliorating or reversing one or more symptoms or side effects of such disease or disorder.

For purposes of this invention, "treatment", "treating", "delaying" or "reversing" further means an approach for obtaining beneficial or desired clinical results, where "beneficial or desired clinical results" include, without limitation, alleviation of a symptom, diminishment of the extent of a disorder or disease, stabilized (i.e., not worsening) disease or disorder state, delay, prevention or slowing of the progression a disease or disorder state, amelioration or palliation of a disease or disorder state, and remission of a disease or disorder, whether partial or total, detectable or undetectable.

In an embodiment, the nutritional supplement of the present invention can be administered to a human subject in need thereof once a day, twice a day, three times a day, four times a day or five times a day.

In an embodiment, the nutritional supplement of the present invention can be administered to a human subject in need thereof daily, every two days, every three days, every four days, every five days, every six days, weekly, bi-weekly, or monthly. In an embodiment, the nutritional supplement of the present invention can be administered daily.

### 3. A method for preparing a nutritional supplement

In the second aspect of this invention, there is provided a method for preparing a nutritional supplement as described herein, wherein the myoglobin is obtained from fermentation by a microorganism. In an embodiment, the myoglobin may be a recombinant myoglobin. In an embodiment, the myoglobin is considered a recombinant myoglobin and is produced by a microorganism which has been genetically modified to express the recombinant myoglobin.

In an embodiment, said method uses a microorganism as described in the first aspect.

In an embodiment, said microorganisms which has been genetically modified to express the recombinant myoglobin comprises a nucleic acid encoding said recombinant myoglobin. These nucleic acids sequence may need to be codon-optimized in order to be expressed by the microorganism.

In an embodiment, the encoded or expressed recombinant myoglobin is derived from steppe mammoth myoglobin (SEQ ID NO: 1). In an embodiment, the recombinant myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 1. A nucleic acid encoding said recombinant myoglobin which is derived from steppe mammoth myoglobin (SEQ ID NO:1) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:15.

In an embodiment, the encoded or expressed recombinant myoglobin is derived from woolly mammoth myoglobin (SEQ ID NO: 2). In an embodiment, the recombinant myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 2. A nucleic acid encoding said recombinant myoglobin which is derived from woolly mammoth myoglobin (SEQ ID NO: 2) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:16.

In an embodiment, the encoded or expressed recombinant myoglobin is derived from pig myoglobin (SEQ ID NO: 3). In an embodiment, the recombinant myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 3. A nucleic acid encoding said recombinant myoglobin which is derived from pig myoglobin (SEQ ID NO: 3) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:17.

In an embodiment, the encoded or expressed recombinant myoglobin is derived from Sheep myoglobin (SEQ ID NO: 4). In an embodiment, the recombinant myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 4. A nucleic acid encoding said recombinant myoglobin which is derived from Sheep myoglobin (SEQ ID NO: 4) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:18.

In an embodiment, the encoded or expressed recombinant myoglobin is derived from cow myoglobin (SEQ ID NO: 5). In an embodiment, the recombinant myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 5. A nucleic acid encoding said recombinant myoglobin which is derived from cow myoglobin (SEQ ID NO: 5) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:19.

In an embodiment, the encoded or expressed recombinant myoglobin is derived from chicken myoglobin (SEQ ID NO: 6). In an embodiment, the recombinant myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 6. A nucleic acid encoding said recombinant myoglobin which is derived from chicken myoglobin (SEQ ID NO: 6) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:20.

In an embodiment, the encoded or expressed recombinant myoglobin is derived from rabbit myoglobin (SEQ ID NO: 7). In an embodiment, the recombinant myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 7. A nucleic acid encoding said recombinant myoglobin which is derived from rabbit myoglobin (SEQ ID NO: 7) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:21.

In an embodiment, the encoded or expressed recombinant myoglobin is derived from bovine myoglobin (SEQ ID NO: 8). In an embodiment, the recombinant myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 8. A nucleic acid encoding said recombinant myoglobin which is derived from bovine myoglobin (SEQ ID NO: 8) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:22.

In an embodiment, the encoded or expressed recombinant myoglobin is derived from mouse myoglobin (SEQ ID NO: 9). In an embodiment, the recombinant myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 9. A nucleic acid encoding said recombinant myoglobin which is derived from mouse myoglobin (SEQ ID NO: 9) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:23.

In an embodiment, the encoded or expressed recombinant myoglobin is derived from rat myoglobin (SEQ ID NO: 10). In an embodiment, the recombinant myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 10. A nucleic acid encoding said recombinant myoglobin which is derived from rat myoglobin (SEQ ID NO: 10) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:24.

In an embodiment, the encoded or expressed recombinant myoglobin is derived from tuna myoglobin (SEQ ID NO: 11). In an embodiment, the recombinant myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 11 . A nucleic acid encoding said recombinant myoglobin which is derived from tuna myoglobin (SEQ ID NO: 11) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:25.

In an embodiment, the encoded or expressed recombinant myoglobin is derived from salmon myoglobin (SEQ ID NO: 12). In an embodiment, the recombinant myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 12. A nucleic acid encoding said recombinant myoglobin which is derived from salmon myoglobin (SEQ ID NO: 12) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:26.

In an embodiment, the encoded or expressed recombinant myoglobin is derived from blue bonito myoglobin (SEQ ID NO: 13). In an embodiment, the recombinant myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 13. A nucleic acid encoding said recombinant myoglobin which is derived from blue bonito myoglobin (SEQ ID NO: 13) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:27.

In an embodiment, the encoded or expressed recombinant myoglobin is derived from sword fish myoglobin (SEQ ID NO: 14). In an embodiment, the recombinant myoglobin comprises a sequence that has at least has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity or similarity with SEQ ID NO: 14. A nucleic acid encoding said recombinant myoglobin which is derived from sword fish myoglobin (SEQ ID NO: 14) may have a sequence that has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO:28.

Cell culturing during the microbial fermentation may be performed for a duration of 14, 13.5, 13, 12.5, 12, 11.5, 11, 10.5, 10, 9.5, 9, 8.5, 8, 7.5, 7, 6.5, 6, 5.5, 5, 4.5, 4, 3.5, 3, 2.5, 2, 1.5 or 1 days, wherein said duration may deviate by 20%. Preferably, cell culturing is performed for a duration of 14, 13.5, 13, 12.5, 12, 11.5, 11, 10.5, 10, 9.5, 9, 8.5, 8, 7.5, 7, 6.5, 6, 5.5, 5, 4.5, 4, 3.5, 3, 2.5, 2, 1.5 or 1 days, wherein said duration may deviate by 10%. More preferably, the cell culturing is performed for 5 days, wherein said duration may deviate by 20%, most preferably by 10%.

The cell culturing will typically result in a production of at least 100 mg/L, 200 mg/L, 300 mg/L, 400 mg/L, 500 mg/L, 600 mg/L, 700 mg/L, 800 mg/L, 900 mg/L, 1 g/L, 2 g/L, 3 g/L, 4 g/L, 5 g/L, 6 g/L, 7 g/L, 8 g/L, 9 g/L, 10 g/L, 11 g/L, 12 g/L, 13 g/L, 14 g/L, 15 g/L, 16 g/L, 17 g/L,18 g/L, 19 g/L, 20 g/L, 21 g/L, 22 g/L, 23 g/L, 24 g/L, 25 g/L, 50 g/L, 75 g/L, 100 g/L, 200 g/L or 300 g/L of a myoglobin, or recombinant myoglobin.

The cell culturing will typically result in at least 10%, 20%, 25%, 30%, 35%, 40%, 45%, 50%, 55%, 60%, 65%, 70%, 75%, 80%, 85%, 90%, 95%, or 100%, preferably at least 20%, more preferably at least 40%, most preferably at least 60% of the carbon source in the growth medium being converted to a myoglobin or recombinant myoglobin.

Cell culturing may also be performed by implementation of a multiple step, preferably a two-step, culture method. For example, a production step of a myoglobin may be preceded by a cellular biomass growth step, wherein only limited production or no production is taking place. The different steps may be carried out using different culture modes and/or different growth media and/or different culture process parameter values, depending on the goal of each step and/or the cultured cell. The biomass during the production step may or may not be actively growing.

The microorganisms used in fermentation to obtain a myoglobin or a recombinant myoglobin, the recovering step and/or purification step as described in the first aspect of the present invention also applies in this second aspect of the invention. In an embodiment, the microorganism is a bacterium, a yeast, or a filamentous fungus, preferably Pichia pastoris.

### 4. General terms

Various embodiments are described herein. Each embodiment as identified herein may be combined together unless otherwise indicated. All patent applications, patents, and printed publications cited herein are incorporated herein by reference in the entireties, except for any definitions, subject matter disclaimers or disavowals, and except to the extent that the incorporated material is inconsistent with the express disclosure herein, in which case the language in this disclosure controls.

One skilled in the art will recognize many methods and materials similar or equivalent to those described herein, which could be used in the practice of the present invention. Indeed, the present invention is in no way limited to the methods and materials described.

In this document and in its claims, the verb "to comprise" and its conjugations is used in its non-limiting sense to mean that items following the word are included, but items not specifically mentioned are not excluded. In addition, the verb "to consist" may be replaced by "to consist essentially of" meaning that a method as described herein may comprise additional step(s) than the ones specifically identified, said additional step(s) not altering the unique characteristic of the invention. In addition, the verb "to consist" may be replaced by "to consist essentially of" meaning that a nutritional supplement, a recombinant myoglobin, a gene construct, a host cell (or methods) as described herein may comprise additional component(s) (or additional steps) than the ones specifically identified, said additional component(s) not altering the unique characteristic of the invention.

Reference to an element by the indefinite article "a" or "an" does not exclude the possibility that more than one of the elements is present, unless the context clearly requires that there be one and only one of the elements. The indefinite article "a" or "an" thus usually means "at least one". As used herein, with "at least" a particular value means that particular value or more. For example, "at least 2" is understood to be the same as "2 or more" i.e., 2, 3, 4, 5, 6, 7, 8, 9, 10, 11, 12, 13, 14, 15, ..., etc.

The terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. The word "about" or "approximately" when used in association with a numerical value (e.g. about 10) preferably means that the value may be the given value (of 10) more or less 0.1% of the value. As used herein, the term "and/or" indicates that one or more of the stated cases may occur, alone or in combination with at least one of the stated cases, up to with all of the stated cases.

In the context of this application, all percentages in the context of a concentration or composition refer to weight percentages, unless defined otherwise. In the context of this application, expressions such as "a parameter having a value of at least X, Y or Z" should be interpreted as said parameter having a value of at least X, of at least Y, or of at least Z.

### Sequence identity

In the context of the invention, a nucleic acid molecule such as a nucleic acid molecule encoding an isolated myoglobin is represented by a nucleic acid or nucleotide sequence which encodes a protein fragment or a polypeptide or a peptide or a derived peptide. It is to be understood that each nucleic acid molecule or protein fragment or polypeptide or peptide or derived peptide or construct as identified herein by a given sequence identity number (SEQ ID NO) is not limited to this specific sequence as disclosed. Each coding sequence as identified herein encodes a given protein fragment or polypeptide or peptide or derived peptide or construct or is itself a protein fragment or polypeptide or construct or peptide or derived peptide.

Throughout this application, each time one refers to a specific nucleotide sequence SEQ ID NO (take SEQ ID NO: X as example) encoding a given protein fragment or polypeptide or peptide or derived peptide, one may replace it by:
i. a nucleotide sequence comprising a nucleotide sequence that has at least 60% sequence identity with SEQ ID NO: X; or
ii. a nucleotide sequence the sequence of which differs from the sequence of a nucleic acid molecule of (i) due to the degeneracy of the genetic code; or
iii. a nucleotide sequence that encodes an amino acid sequence that has at least 60% amino acid identity or similarity with an amino acid sequence encoded by a nucleotide sequence SEQ ID NO: X.

Another preferred level of sequence identity or similarity is 70%. Another preferred level of sequence identity or similarity is 75%. Another preferred level of sequence identity or similarity is 80%. Another preferred level of sequence identity or similarity is 85%. Another preferred level of sequence identity or similarity is 90%. Another preferred level of sequence identity or similarity is 95%. Another preferred level of sequence identity or similarity is 99%.

Throughout this application, each time one refers to a specific amino acid sequence SEQ ID NO (take SEQ ID NO: Y as example), one may replace it by: a polypeptide represented by an amino acid sequence comprising a sequence that has at least 60% sequence identity or similarity with amino acid sequence SEQ ID NO: Y. Another preferred level of sequence identity or similarity is 70%. Another preferred level of sequence identity or similarity is 75%. Another preferred level of sequence identity or similarity is 80%. Another preferred level of sequence identity or similarity is 85%. Another preferred level of sequence identity or similarity is 90%. Another preferred level of sequence identity or similarity is 95%. Another preferred level of sequence identity or similarity is 99%.

Each nucleotide sequence or amino acid sequence described herein by virtue of its identity or similarity percentage with a given nucleotide sequence or amino acid sequence respectively has in a further preferred embodiment an identity or a similarity of at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% with the given nucleotide or amino acid sequence, respectively.

The terms "homology", "sequence identity" and the like are used interchangeably herein. Sequence identity is described herein as a relationship between two or more amino acid (polypeptide or protein) sequences or two or more nucleic acid (polynucleotide) sequences, as determined by comparing the sequences. In a preferred embodiment, sequence identity is calculated based on the full length of two given SEQ ID NO's or on a part thereof. Part thereof preferably means at least 50%, 60%, 70%, 80%, 90%, or 100% of both SEQ ID NO's. In the art, "identity" also refers to the degree of sequence relatedness between amino acid or nucleic acid sequences, as the case may be, as determined by the match between strings of such sequences. "Similarity" between two amino acid sequences is determined by comparing the amino acid sequence and its conserved amino acid substitutes of one polypeptide to the sequence of a second polypeptide. "Identity" and "similarity" can be readily calculated by known methods, including but not limited to those described in Bioinformatics and the Cell: Modern Computational Approaches in Genomics, Proteomics and transcriptomics, Xia X., Springer International Publishing, New York, 2018; and Bioinformatics: Sequence and Genome Analysis, Mount D., Cold Spring Harbor Laboratory Press, New York, 2004, each incorporated herein by reference.

"Sequence identity" and "sequence similarity" can be determined by alignment of two peptide or two nucleotide sequences using global or local alignment algorithms, depending on the length of the two sequences. Sequences of similar lengths are preferably aligned using a global alignment algorithms (e.g. Needleman-Wunsch) which aligns the sequences optimally over the entire length, while sequences of substantially different lengths are preferably aligned using a local alignment algorithm (e.g. Smith-Waterman). Sequences may then be referred to as "substantially identical" or "essentially similar" when they (when optimally aligned by for example the program EMBOSS needle or EMBOSS water using default parameters) share at least a certain minimal percentage of sequence identity (as described below).

A global alignment is suitably used to determine sequence identity when the two sequences have similar lengths. When sequences have a substantially different overall length, local alignments, such as those using the Smith-Waterman algorithm, are preferred. EMBOSS needle uses the Needleman-Wunsch global alignment algorithm to align two sequences over their entire length (full length), maximizing the number of matches and minimizing the number of gaps. EMBOSS water uses the Smith-Waterman local alignment algorithm. Generally, the EMBOSS needle and EMBOSS water default parameters are used, with a gap open penalty = 10 (nucleotide sequences) / 10 (proteins) and gap extension penalty = 0.5 (nucleotide sequences) / 0.5 (proteins). For nucleotide sequences the default scoring matrix used is DNAfull and for proteins the default scoring matrix is Blosum62 (Henikoff & Henikoff, 1992, PNAS 89, 915-919, incorporated herein by reference).

Alternatively, percentage similarity or identity may be determined by searching against public databases, using algorithms such as FASTA, BLAST, etc. Thus, the nucleic acid and protein sequences of some embodiments of the present invention can further be used as a "query sequence" to perform a search against public databases to, for example, identify other family members or related sequences. Such searches can be performed using the BLASTn and BLASTx programs (version 2.0) of Altschul, et al. (1990) J. Mol. Biol. 215:403-10, incorporated herein by reference. BLAST nucleotide searches can be performed with the NBLAST program, score = 100, wordlength = 12 to obtain nucleotide sequences homologous to oxidoreductase nucleic acid molecules of the invention. BLAST protein searches can be performed with the BLASTx program, score = 50, wordlength = 3 to obtain amino acid sequences homologous to protein molecules of the invention. To obtain gapped alignments for comparison purposes, Gapped BLAST can be utilized as described in Altschul et al., (1997) Nucleic Acids Res. 25(17): 3389-3402, incorporated herein by reference. When utilizing BLAST and Gapped BLAST programs, the default parameters of the respective programs (e.g., BLASTx and BLASTn) can be used. See the homepage of the National Center for Biotechnology Information accessible on the world wide web at www.ncbi.nlm.nih.gov/.

Optionally, in determining the degree of amino acid similarity, the skilled person may also take into account so-called conservative amino acid substitutions. As used herein, "conservative" amino acid substitutions refer to the interchangeability of residues having similar side chains. Examples of classes of amino acid residues for conservative substitutions are given in the Tables below.

| | |
|---|---|
| Acidic Residues | Asp (D) and Glu (E) |
| Basic Residues | Lys (K), Arg (R), and His (H) |
| Hydrophilic Uncharged Residues | Ser (S), Thr (T), Asn (N), and Gln (Q) |
| Aliphatic Uncharged Residues | Gly (G), Ala (A), Val (V), Leu (L), and Ile (I) |
| Non-polar Uncharged Residues | Cys (C), Met (M), and Pro (P) |
| Aromatic Residues | Phe (F), Tyr (Y), and Trp (W) |

Alternative conservative amino acid residue substitution classes:

| | | | |
|---|---|---|---|
| 1 | A | S | T |
| 2 | D | E | |
| 3 | N | Q | |
| 4 | R | K | |
| 5 | I | L | M |
| 6 | F | Y | W |

Alternative physical and functional classifications of amino acid residues:

| | |
|---|---|
| Alcohol group-containing residues | S and T |
| Aliphatic residues | I, L, V, and M |
| Cycloalkenyl-associated residues | F, H, W, and Y |
| Hydrophobic residues | A, C, F, G, H, I, L, M, R, T, V, W, and Y |
| Negatively charged residues | D and E |
| Polar residues | C, D, E, H, K, N, Q, R, S, and T |
| Positively charged residues | H, K, and R |
| Small residues | A, C, D, G, N, P, S, T, and V |
| Very small residues | A, G, and S |
| Residues involved in turn formation | A, C, D, E, G, H, K, N, Q, R, S, P and T |
| Flexible residues | Q, T, K, S, G, P, D, E, and R |

For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to Ser; Arg to Lys; Asn to Gln or His; Asp to Glu; Cys to Ser or Ala; Gln to Asn; Glu to Asp; Gly to Pro; His to Asn or Gln; lie to Leu or Val; Leu to lie or Val; Lys to Arg; Gln or Glu; Met to Leu or Ile; Phe to Met, Leu or Tyr; Ser to Thr; Thr to Ser; Trp to Tyr; Tyr to Trp or Phe; and, Val to lie or Leu.

### Proteins and amino acids

The terms "protein" or "polypeptide" or "amino acid sequence" are used interchangeably and refer to molecules consisting of a chain of amino acids, without reference to a specific mode of action, size, 3-dimensional structure or origin. In amino acid sequences as described herein, amino acids or "residues" are denoted by three-letter symbols. These three-letter symbols as well as the corresponding one-letter symbols are well known to a person of skill in the art and have the following meaning: A (Ala) is alanine, C (Cys) is cysteine, D (Asp) is aspartic acid, E (Glu) is glutamic acid, F (Phe) is phenylalanine, G (Gly) is glycine, H (His) is histidine, I (Ile) is isoleucine, K (Lys) is lysine, L (Leu) is leucine, M (Met) is methionine, N (Asn) is asparagine, P (Pro) is proline, Q (Gln) is glutamine, R (Arg) is arginine, S (Ser) is serine, T (Thr) is threonine, V (Val) is valine, W (Trp) is tryptophan, Y (Tyr) is tyrosine. A residue may be any proteinogenic amino acid, but also any non-proteinogenic amino acid such as D-amino acids and modified amino acids formed by post-translational modifications, and also any non-natural amino acid, as described herein..

### Gene or coding sequence

The term "gene" means a DNA fragment comprising a region (transcribed region), which is transcribed into an RNA molecule (e.g. an mRNA) in a cell, operably linked to suitable regulatory regions (e.g. a promoter). A gene will usually comprise several operably linked fragments, such as a promoter, a 5' leader sequence, a coding region and a 3'-nontranslated sequence (3'-end) e.g. comprising a polyadenylation- and/or transcription termination site. "Expression of a gene" refers to the process wherein a DNA region which is operably linked to appropriate regulatory regions, particularly a promoter, is transcribed into an RNA, which is biologically active, i.e. which is capable of being translated into a biologically active protein or peptide. As used herein, a "regulator" or "transcriptional regulator" is a protein that controls the rate of transcription of genetic information from DNA to messenger RNA, by binding to a specific DNA sequence.

### Promoter

As used herein, the term "promoter" or "transcription regulatory sequence" refers to a nucleic acid fragment that functions to control the transcription of one or more coding sequences, and is located upstream with respect to the direction of transcription of the transcription initiation site of the coding sequence, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter. A "constitutive" promoter is a promoter that is active under most physiological and developmental conditions. An "inducible" and/or "repressible" promoter is a promoter that is physiologically or developmentally regulated to be induced and/or repressed, e.g. by the application of a chemical inducer or repressing signal.

As used herein, the term "operably linked" refers to a linkage of polynucleotide elements in a functional relationship. A nucleic acid is "operably linked" when it is placed into a functional relationship with another nucleic acid sequence. For instance, a transcription regulatory sequence is operably linked to a coding sequence if it affects the transcription of the coding sequence. Operably linked means that the DNA sequences being linked are typically contiguous and, where necessary to join two protein encoding regions, contiguous and in reading frame. Linking can be accomplished by ligation at convenient restriction sites or at adapters or linkers inserted in lieu thereof, or by gene synthesis.

### Gene constructs and expression vectors

Gene constructs as described herein could be prepared using any cloning and/or recombinant DNA techniques, as known to a person of skill in the art, in which a nucleotide sequence encoding said isolated myoglobin is expressed in a suitable cell, e.g. cultured cells or cells of a multicellular organism, such as described in Ausubel et al., "Current Protocols in Molecular Biology", Greene Publishing and Wiley-Interscience, New York (1987) and in Sambrook and Russell (2001, *supra*); both of which are incorporated herein by reference in their entirety. Also see, Kunkel (1985) Proc. Natl. Acad. Sci. 82:488 (describing site directed mutagenesis) and Roberts et al. (1987) Nature 328:731-734 or Wells, J.A., et al. (1985) Gene 34: 315 (describing cassette mutagenesis).

The phrase "expression vector" or "vector" generally refers to a tool in molecular biology used to obtain gene expression in a cell, for example by introducing a nucleotide sequence that is capable of effecting expression of a gene or a coding sequence in a host compatible with such sequences. An expression vector carries a genome that is able to stabilize and remain episomal in a cell. Within the context of the invention, a cell may mean to encompass a cell used to make the construct or a cell wherein the construct will be administered. Alternatively, a vector is capable of integrating into a cell's genome, for example through homologous recombination or otherwise.

These expression vectors typically include at least suitable promoter sequences and optionally, transcription termination signals. An additional factor necessary or helpful in effecting expression can also be used as described herein. A nucleic acid or DNA or nucleotide sequence encoding a myoglobin is incorporated into a DNA construct capable of introduction into and expression in an *in vitro* cell culture. Specifically, a DNA construct is suitable for replication in a prokaryotic host, such as bacteria, *e.g*., *E. coli,* or can be introduced into a cultured mammalian, plant, insect, (*e.g*., Sf9), yeast, fungi or other eukaryotic cell lines.

A DNA construct prepared for introduction into a particular host may include a replication system recognized by the host, an intended DNA segment encoding a desired polypeptide, and transcriptional and translational initiation and termination regulatory sequences operably linked to the polypeptide-encoding segment. The term "operably linked" has already been described herein. For example, a promoter or enhancer is operably linked to a coding sequence if it stimulates the transcription of the sequence. DNA for a signal sequence is operably linked to DNA encoding a polypeptide if it is expressed as a preprotein that participates in the secretion of a polypeptide. Generally, a DNA sequence that is operably linked are contiguous, and, in the case of a signal sequence, both contiguous and in reading frame. However, enhancers need not be contiguous with a coding sequence whose transcription they control. Linking is accomplished by ligation at convenient restriction sites or at adapters or linkers inserted in lieu thereof, or by gene synthesis.

The selection of an appropriate promoter sequence generally depends upon the host cell selected for the expression of a DNA segment. Examples of suitable promoter sequences include prokaryotic, and eukaryotic promoters well known in the art (see, *e.g.* Sambrook and Russell, 2001, *supra*)*.* A transcriptional regulatory sequence typically includes a heterologous enhancer or promoter that is recognised by the host. The selection of an appropriate promoter depends upon the host, but promoters such as the trp, lac and phage promoters, tRNA promoters and glycolytic enzyme promoters are known and available (see, *e.g*. Sambrook and Russell, 2001, *supra*)*.* An expression vector includes the replication system and transcriptional and translational regulatory sequences together with the insertion site for the polypeptide encoding segment. In most cases, the replication system is only functional in the cell that is used to make the vector (bacterial cell as *E*. *Coli*)*.* Most plasmids and vectors do not replicate in the cells infected with the vector. Examples of workable combinations of cell lines and expression vectors are described in Sambrook and Russell (2001, *supra*) and in Metzger et al. (1988) Nature 334: 31-36. For example, suitable expression vectors can be expressed in, yeast, *e.g. S*. *cerevisiae, e.g.,* insect cells, *e.g*., Sf9 cells, mammalian cells, *e.g*., CHO cells and bacterial cells, *e.g.*, *E. coli.* A cell may thus be a prokaryotic or eukaryotic host cell. A cell may be a cell that is suitable for culture in liquid or on solid media. Alternatively, a host cell is a cell that is part of a multicellular organism such as a transgenic plant or animal.

For example if a bacterium (preferably E. coli) is used as host cell, the following regulatory regions may be used. A promoter suitable to be used in a bacterium is lac, trp, tac, T7, phoA, ara, xapA, cad, recA, spc, bla, P1 and P2 from rrnB ribosomal RNA operon, PL promoter from phage λ. A terminator suitable to be used in a bacterium is lac, trp, tac, T7 (used in example), phoA, ara, xapA, cad, recA, spc, bla, P1 and P2 from rrnB ribosomal RNA operon, PL terminator from phage λ. A preferred promoter used is a T7 promoter and/or a preferred terminator is the T7 terminator. A preferred signal peptide for excretion is E. coli Sec-recognition peptide (SecA), E. coli Tet-recognition peptide, E. coli dsbA, E. coli phoA, E. coli pelB, E. coli MBP (maltose binding protein). A marker suitable for E coli is ampicillin. Alternatively, the proBA operon from E. coli strain K12, including its original transcription regulatory elements may be used to facilitate selection without antibiotics.

In some embodiments, the microorganisms do not comprise a selection marker or an antibiotic resistance marker. In an embodiment, the microorganism is a yeast. In an embodiment, the yeast is *Pichia pastoris.*

In another example, if a yeast is used as host cell, the following regulatory regions may be used. A promoter suitable to be used in yeast may be a constitutive promoter. Examples of suitable constitutive promoters include: a glycolytic promoter selected from FBA1, TPI1, PGK1, PYK1, TDH3, ENO2, HXK2, PGI1, PFK1, PFK2, GPM1 gene or a non-glycolytic promoter of the TEF2 gene. A suitable promoter to be used in yeast may be inducible. If the yeast is a *Pichia,* the methanol inducible promoter AOX1 is preferred. Otherwise the GAL1 promoter (galactose-inducible) may be used when the yeast is *S*. *cerevisiae.* The genes mentioned from which a promoter could be derived for a yeast as host cell could also be used to derive a terminator for the same yeast. A preferred signal peptide for excretion for *Pichia* (and *Saccharomyces*) includes: the *S*. *cerevisiae* alpha mating factor pre- pro- secretion signal peptide, the *S. cerevisiae* Ost1 signal peptide, the *S*. *cerevisiae* Aga2 signal peptide and fusions thereof.

In another example, if a filamentous fungus is used as host cell, the following regulatory regions may be used. The following promoters may be used: the *Aspergillus niger* glucoamylase promoter (*glaA*), the *Aspergillus nidulans* alcohol dehydrogenase promoter (*alcA*) or the *Aspergillus oryzae* taka-amylase A promoter (*amyB*)*,* the *Aspergillus niger* alcohol dehydrogenase promoter (*adhA*)*,* the *Trichoderma reesei* pyruvate kinase promoter (*pki*) or the *Aspergillus nidulas* glyceraldehyde-3-phosphate dehydrogenase promoter (*gpdA*)*.* The genes mentioned from which a promoter could be derived for a filamentous fungus as host cell could also be used to derive a terminator for the same filamentous fungus. A preferred signal peptide for excretion for a filamentous fungus, preferably an *Aspergillus* includes: the *Aspergillus niger* glucoamylase signal peptide (*glaA*), the *Aspergillus niger* a-galactosidase signal peptide (*AglB*) and the *Trichoderma reesei* cellobiohydrolase I (*CbhI*)*.* A preferred promoter and terminator for *Aspergillus* (more preferably for *Aspergillus niger*) are the glucoamylase promoter and the glucoamylase terminator of *Aspergillus niger.*

Gene constructs described herein can be placed in expression vectors. Thus, in another aspect there is provided an expression vector comprising a gene construct as described in any of the preceding embodiments.

Expression may be assessed by any method known to a person of skill in the art. For example, expression may be assessed by measuring the levels of transgene expression in the transduced tissue on the level of the mRNA or the protein by standard assays known to a person of skill in the art, such as qPCR, RNA sequencing, Northern blot analysis, Western blot analysis, mass spectrometry analysis of protein-derived peptides or ELISA. Expression may be assessed at any time after administration of the gene construct, expression vector or composition as described herein. In some embodiments herein, expression may be assessed after 1 week, 2 weeks, 3 weeks, 4, weeks, 5 weeks, 6 weeks, 7 weeks, 8 weeks, 9, weeks, 10 weeks, 11 weeks, 12 weeks, 14 weeks, 16 weeks, 18 weeks, 20 weeks, 22 weeks, 24 weeks, 28 weeks, 32 weeks, 36 weeks, 40 weeks, or more.

Suitable cell culturing methods for use in the process of producing recombinant protein are known to the skilled person, for example, in van't Riet, K. and Tramper, J., 1st edition, Basic Bioreactor Design, CRC Press, NY, 1991. Such methods include, but are not limited to, submerged fermentation in liquid media, surface fermentation on liquid media and solid-state fermentations. Cell culturing may, for example, be performed by cultivation in micro-titer plates, shake-flasks, small-scale benchtop bioreactors, medium-scale bioreactors and/or large-scale bioreactors in a laboratory and/or an industrial setting. Suitable cell culturing modes include, but are not limited to, continuous, batch and/or fed-batch fermentation as well as their combinations. Cell culturing may be performed using continuous fermentation, batch fermentation, preferably fed batch fermentation.

In the context of the invention, "culture medium", hereinafter alternately referred to as "growth medium", can be interpreted to encompass cases wherein the cultured cells are absent as well as cases wherein the cultured cells are present in the culture medium. "Culture broth" refers to the culture medium wherein the cultured cells are present. "Culture supernatant" refers to the culture medium wherein the cultured cells are absent. "Cell-free extract" refers to a cell lysate not comprising the cellular debris. Cell culturing as part of the process of the invention can be performed under conditions conducive to the production of the introduced myoglobins, which are known to the skilled person. Such conditions depend not only on the chemical composition of the culture medium but also on other process parameters including culture duration, temperature, O₂ levels in the culture broth and/or headspace, CO₂ levels in the culture broth and/or headspace, pH, ionic strength, agitation speed, hydrostatic pressure and the like. Cell culturing can take place using a culture medium comprising suitable nutrients, such as carbon and nitrogen sources and additional compounds such as inorganic salts and vitamins, using procedures known in the art (see, e. g. Bennett, W. and Lasure, L., 1st edition, More Gene Manipulations in Fungi, Academic Press, CA, 1991). Suitable growth media are available from commercial suppliers or may be prepared using published compositions that are suitable for the respective hosts (e.g. in catalogues of the Centraalbureau Voor Schimmelcultures collection (CBS) or of the American Type Culture Collection (ATCC)).

The exact composition of the growth medium and the values of culture process parameters are not critical features of the invention. Any growth medium composition may be contemplated, as long as it allows for growth of the host cell and production of the introduced myoglobins. The growth medium will typically comprise a carbon source to be used for the growth of the cultured cell. The skilled person understands that suitable carbon sources may be added externally to the growth medium or may already be present in said medium. Carbon sources may be present or added individually or in mixtures of multiple carbon sources. Examples of suitable carbon sources known in the art include simple sugars such as glucose, glycerol, maltose, sucrose, xylose, arabinose, complex sugars such as maltodextrins, hydrolysed starch, starch, molasses, and second-generation feedstocks. Second-generation feedstocks can be particularly attractive because of their lower carbon footprint. Second-generation feedstocks will typically comprise lignocellulosic material. Such material includes any lignocellulose and/or hemicellulose-based materials. Such material may be sourced from agricultural, industrial or municipal, preferably agricultural, waste streams. Examples of suitable materials include (agricultural) biomass, commercial organic matter, municipal solid waste, virgin biomass such as waste paper and garden waste, or non-virgin biomass. General forms of biomass include trees, shrubs and pastures, wheat, wheat straw, sugarcane bagasse, switchgrass, Japanese pampas grass, corn, corn stover, corn cob, canola stalk, soybean stalk, sweet corn, corn kernels, products and by-products from cereal milling (including wet milling and dry milling), such as corn, wheat, and barley, often referred to as "bran or fiber", and municipal solids. Biomass can also be grassy materials, agricultural residues, forestry residues, municipal solid waste, waste paper, and pulp and paper mill residues. Agricultural biomass includes branches, shrubs, tows, corn and corn straw, energy crops, forests, fruits, flowers, cereals, pastures, herbaceous crops, leaves, bark, needles, logs, roots, young trees, short term rotating woody crops, shrubs, switch herbs, trees, vegetables, fruits, vines, sugar beet pulp, wheat middlings, oat hulls, and hard and soft timber (not including toxic wood), and organic waste materials resulting from agricultural processes including agriculture and forestry activities, particularly forestry wood waste. Agricultural biomass may be any of the foregoing alone, or any combination or mixture thereof. Carbon sources such as organic acids, aldehydes, ketones, esters and alcohols may also be contemplated. The use of growth media comprising combinations of multiple different carbon sources may also be contemplated in the process of the invention. Such media could, as a non-limiting example, combine more oxidized carbon sources such as organic acids with more reduced carbon sources such as alcohols. Examples of suitable nitrogen sources known in the art include soy bean meal, corn steep liquor, yeast extract, whey protein, egg protein, casein hydrolysate, urea, ammonia, ammonium salts and nitrate salts. Examples of additional suitable compounds known in the art include phosphate, sulphate, metals such as magnesium, trace elements and vitamins. The exact growth medium requirements will vary based on the host cell, e.g. between yeasts, bacteria and filamentous fungi, said requirements will be known to the skilled person. Accordingly, the growth medium may be a complete (rich) medium or a minimal medium, i.e. a medium comprising only the absolutely necessary components for growth depending on the cultured host cell.

Similar to the composition of the growth medium, process parameters can be assigned any value, as long as they allow for growth of the host cell and production of the introduced myoglobins. Typically, said values will differ based on the host cell that is being cultured and will be known to the skilled person. Preferably, the process according to the invention is an oxygen-limited or aerobic process, meaning that cell culturing is performed under oxygen-limited or aerobic conditions, more preferably the process is oxygen-limited. Oxygen-limited conditions, also known as a micro-aerobic conditions, are culture conditions in which the oxygen consumption is limited by the availability of oxygen. The degree of oxygen limitation is determined by the amount and composition of the ingoing gas flow as well as the actual mixing/mass transfer properties of the fermentation equipment used. Preferably, under oxygen-limited conditions in a liquid culture, the rate of oxygen consumption is at least about 5.5 mmol/L/h, more preferably at least about 6 mmol/L/h and even more preferably at least about 7 mmol/L/h. Aerobic conditions are culture conditions in which the oxygen consumption is not limited by the availability of oxygen.

Cell culturing may be performed at a temperature value that is optimal for the cell, typically at a temperature range of 16-42 °C. In some embodiments, the temperature ranges between 20-40 °C, more preferably between 25-38 °C, most preferably between 28-36 °C. In some most preferred embodiments, a temperature value of about 30 or 36 °C is used.

Cell culturing may be performed at a pH value that is optimal for the cell. In some embodiments, the culture pH value is about pH 2.5, about pH 3.0, about pH 3.5, about pH 4.0, about pH 4.5, about pH 5, about pH 5.5, about pH 6, about pH 6.5, about pH 7, about pH 7.5, about pH 8.0, about pH 8.5, about pH 9. In preferred embodiments, the pH ranges from about pH 3.0 to about pH 9, more preferably from about pH 3.5 to pH 7. In some most preferred embodiments, a pH value of about 6 is used.

Cell culturing may be performed at an ionic strength value of the culture medium that is optimal for the cell, typically at a range between 50 mM - 2 M. In some embodiments, the ionic strength of the culture medium ranges between 75 mM - 1 M, more preferably between 100 mM - 750 mM. In some most preferred embodiments, an ionic strength value of about 100 mM is used.

### Overview of the sequence listing

| Protein | SEQ ID NO protein | SEQ ID NO coding nucleic acid |
|---|---|---|
| Steppe mammoth myoglobin | 1 | 15 |
| Woolly mammoth myoglobin | 2 | 16 |
| Pig myoglobin | 3 | 17 |
| Sheep myoglobin | 4 | 18 |
| Cow myoglobin | 5 | 19 |
| Chicken myoglobin | 6 | 20 |
| Rabbit myoglobin | 7 | 21 |
| Bovine myoglobin | 8 | 22 |
| Mouse myoglobin | 9 | 23 |
| Rat myoglobin | 10 | 24 |
| Tuna myoglobin | 11 | 25 |
| Salmon myoglobin | 12 | 26 |
| Blue bonito myoglobin | 13 | 27 |
| Sword fish myoglobin | 14 | 28 |

### Detailed sequences

- SEQ ID NO:1 Steppe mammoth myoglobin 
MGLSDGEWELVLKTWGKVEADIPGHGLEVFVRLFTGHPETLEKFDKFKHLKTEGEMKASEDLKK
QGVTVLTALGGILKKKGHHQAEIQPLAHSHATKHKIPIKYLEFISDAIIHVLQSKHPAEFGADAQGA
MKKALELFRNDIAAKYKELGFQG
- SEQ ID NO:2 Woolly mammoth myoglobin 
MGLSDGEWELVLKTWGKVEADIPGHGLEVFVRLFTGHPETLEKFDKFKHLKTEGEMKASEDLKK
QGVTVLTALGGILKKKGHHQAEIQPLAQSHATKHKIPIKYLEFISDAIIHVLQSKHPAEFGADAQGA
MKKALELFRNDIAAKYKELGFQG
- SEQ ID NO:3 Pig myoglobin 
MGLSDGEWQLVLNVWGKVEADVAGHGQEVLIRLFKGHPETLEKFDKFKHLKSEDEMKASEDLK
KHGNTVLTALGGILKKKGHHEAELTPLAQSHATKHKIPVKYLEFISEAIIQVLQSKHPGDFGADAQ
GAMSKALELFRNDMAAKYKELGFQG
- SEQ ID NO:4 Sheep myoglobin 
MGLSDGEWQLVLNAWGKVEADVAGHGQEVLIRLFTGHPETLEKFDKFKHLKTEAEMKASEDLK
KHGNTVLTALGGILKKKGHHEAEVKHLAESHANKHKIPVKYLEFISDAIIHVLHAKHPSDFGADAQ
GAMSKALELFRNDMAAQYKVLGFQG
- SEQ ID NO:5 Cow myoglobin 
MGLSDGEWQLVLNAWGKVEADVAGHGQEVLIRLFTGHPETLEKFDKFKHLKTEAEMKASEDLK
KHGNTVLTALGGILKKKGHHEAEVKHLAESHANKHKIPVKYLEFISDAIIHVLHAKHPSDFGADAQA
AMSKALELFRNDMAAQYKVLGFHG
- SEQ ID NO:6 Chicken myoglobin 
MGLSDQEWQQVLTIWGKVEADIAGHGHEVLMRLFHDHPETLDRFDKFKGLKTPDQMKGSEDLK
KHGATVLTQLGKILKQKGNHESELKPLAQTHATKHKIPVKYLEFISEVIIKVIAEKHAADFGADSQA
AMKKALELFRNDMASKYKEFGFQG
- SEQ ID NO:7 Rabbit myoglobin 
MGLSDAEWQLVLNVWGKVEADLAGHGQEVLIRLFHTHPETLEKFDKFKHLKSEDEMKASEDLKK
HGNTVLTALGAILKKKGHHEAEIKPLAQSHATKHKIPVKYLEFISEAIIHVLHSKHPGDFGADAQAA
MSKALELFRNDIAAQYKELGFQG
- SEQ ID NO:8 Bovine myoglobin 
MGLSDGEWQLVLNAWGKVEADVAGHGQEVLIRLFTGHPETLEKFDKFKHLKTEAEMKASEDLK
KHGNTVLTALGGILKKKGHHEAEVKHLAESHANKHKIPVKYLEFISDAIIHVLHAKHPSDFGADAQA
AMSKALELFRNDMAAQYKVLGFHG
- SEQ ID NO:9 Mouse myoglobin 
MGLSDGEWQLVLNVWGKVEADLAGHGQEVLIGLFKTHPETLDKFDKFKNLKSEEDMKGSEDLK
KHGCTVLTALGTILKKKGQHAAEIQPLAQSHATKHKIPVKYLEFISEIIIEVLKKRHSGDFGADAQGA
MSKALELFRNDIAAKYKELGFQG
- SEQ ID NO:10 Rat myoglobin 
MGLSDGEWQMVLNIWGKVEGDLAGHGQEVLISLFKAHPETLEKFDKFKNLKSEEEMKSSEDLKK
HGCTVLTALGTILKKKGQHAAEIQPLAQSHATKHKIPVKYLEFISEVIIQVLKKRYSGDFGADAQGA
MSKALELFRNDIAAKYKELGFQG
- SEQ ID NO:11 Tuna myoglobin 
MADFDAVLKCWGPVEADYTTIGGLVLTRLFKEHPETQKLFPKFAGIAQADIAGNAAVSAHGATVL
KKLGELLKAKGSHAAILKPLANSHATKHKIPINNFKLISEVLVKVMHEKAGLDAGGQTALRNVMGIII
ADLEANYKELGFSG
- SEQ ID NO:12 Salmon myoglobin 
MANYDMVLQCWEPVEADYNNHGGLVLSRLFAEHPETLTLFPKFAGIAAGDLSGNAAVAAHGATV
LRKLGELLNARGDHAATLKSLATTHANKHKIPLKNFTLITNIICKVMGEKAGLDEAGQEALRQVMG
VIIADINVTYMELGFAG
- SEQ ID NO:13 Blue bonito myoglobin 
ADFDAVLKFWGPVEADYTSHGGLVLTRLFKEHPETQKLFPKFTGIAQADMAGNAAISAH
GATVLKKLGELLKAKGNHAAILKPMANSHATKHKIPINNFKLISEIIVKVMQEKAGMDAG
GQQALRNVMAAVIADLEANYKELGFSG
- SEQ ID NO:14 Sword fish myoglobin

MAEFEVVLKHWGPVEADYASHGNLVLTRLFTEHPDTQKLFPKFAGIAQGDMAGNAAISAHGATV
LKKLGELLKAKGNHATTLKALATTHATKHKIPIKNFELIAEVIGKVMEEKAGLDSVGREALRDVMAR
VIADIDATYKELGFTG

## Claims

1. A nutritional supplement comprising a myoglobin for humans at risk for developing iron deficiency or an iron deficiency-related disorder.

2. The nutritional supplement according to claim 1, wherein the myoglobin is a myoglobin obtained from microbial fermentation.

3. The nutritional supplement according to claim 2, wherein said supplement comprises at least part of the fermentation broth or part of the microbial host.

4. The nutritional supplement according to claim 2 or 3, wherein myoglobin is extracellularly secreted from the microbial host, and/or wherein the myoglobin is recombinant myoglobin.

5. The nutritional supplement according to any one of claims 1-4, wherein the myoglobin is from steppe mammoth, woolly mammoth, pig, sheep, cow, chicken, rabbit, bovine, mouse, rat, tuna, salmon, blue bonito, or sword fish.

6. The nutritional supplement according to any one of claims 1-5, wherein the myoglobin is represented by a sequence having at least 95% identity with any one of SEQ ID NO: 1 to 14.

7. The nutritional supplement according to any one of claims 1-6, wherein the nutritional supplement comprises 0.01 mg to 18 mg of Fe expressed as myoglobin.

8. The nutritional supplement according to any one of claims 1-7, wherein the nutritional supplement comprises at least 0.01 % by weight of the myoglobin.

9. The nutritional supplement according to any one of claims 1-8, wherein the nutritional supplement is for oral administration.

10. The nutritional supplement according to any one of claims 1-9, wherein the nutritional supplement is formulated as a powder, a capsule, a tablet, a chewable, an extruded bar, or a liquid solution or suspension.

11. The nutritional supplement according to any one of claims 1-10, wherein the nutritional supplement further comprises one or more food-grade additives selected from the group comprising: mixing aids, emulsifiers, sweeteners, preservatives, colorants, and flavour additives.

12. The nutritional supplement according to any one of claims 1-11 for use as a medicament, preferably comprises a pharmaceutically acceptable carrier.

13. The nutritional supplement according to any one of claims 1-12 for use in the treatment of iron deficiency or an iron deficiency-related disorder.

14. A method for preparing a nutritional supplement according to any one of claims 1-10, wherein the myoglobin is a myoglobin obtained from fermentation by a microorganism, and/or wherein the myoglobin is recombinant myoglobin.

15. A method for a nutritional supplement according to claim 14, wherein the microorganism is a bacterium, a yeast, or a filamentous fungus, preferably Pichia pastoris.
